# EUROPÄISCHE PATENTSCHRIFT

(11) **EP 2 726 026 B1**
(45) Veröffentlichungstag und Bekanntmachung des Hinweises auf die Patenterteilung: **05.09.2018**
(21) Anmeldenummer: 12728202.8
(22) Anmeldetag: 12.06.2012
(51) Int. Cl.: A61F 2/958

(54) **BALLONKATHETER ZUR STENT-EINFÜHRUNG**
BALLOON CATHETER FOR INSERTING A STENT
CATHÉTER À BALLONNET POUR INSERTION D'ENDOPROTHÈSE

(30) Priorität: 28.06.2011 US 201161501796 P
(43) Veröffentlichungstag der Anmeldung: 07.05.2014
(73) Patentinhaber: Biotronik AG, 8180 Bülach (CH)
(72) Erfinder: WESSELMANN, Matthias, 8455 Rüdlingen (CH); ECKERMANN, Fabian, 8001 Zürich (CH); HANI, Semijan, 8180 Bülach (CH); PFENNINGER-GANZ, Susanne, 8607 Aathal (CH); LANG, Hans, 8107 Buchs (CH); CONZELMANN, Tommy, 8200 Schaffhausen (CH); QUINT, Bodo, 8154 Oberglatt (CH)
(74) Vertreter: Galander, Marcus
(86) Internationale Anmeldenummer: PCT/EP2012/061060
(87) Internationale Veröffentlichungsnummer: WO 2013/000699

(56) Entgegenhaltungen:
- EP-A1- 0 974 315
- EP-A1- 0 982 010
- WO-A1-00/45740
- DE-C1- 19 833 215
- US-A- 5 766 203
- US-A- 6 077 273
- US-A1- 2006 253 185

## Beschreibung

Die Erfindung betrifft eine Stent-Einführanordnung mit einem Ballonkatheter als Tragstruktur und einem hierauf mit einer Crimp-Verbindung fixierten Stent. Sie betrifft des Weiteren einen Ballonkatheter zur Bildung einer solchen Stent-Einführanordnung.

Implantate zum Offenhalten von Hohlorganen, sog. Gefäßstützen (Stents), werden seit längerem zum Verhindern eines erneuten Verschlusses von Blutgefäßen, speziell von Herzkranzgefäßen, nach deren Aufdehnung eingesetzt, aber auch, um durch Tumore verursachte Verengungen der Luftröhre oder Speiseröhre oder von Gallenwegen nach einer Aufdehnung offen zu halten. Sie werden mittels eines Ballonkatheters in das offen zu haltende Hohlorgan eingeführt und dort an der vorgesehenen Stelle durch Expandieren des Ballons auf den beabsichtigten Durchmesser aufgeweitet und zugleich fixiert.

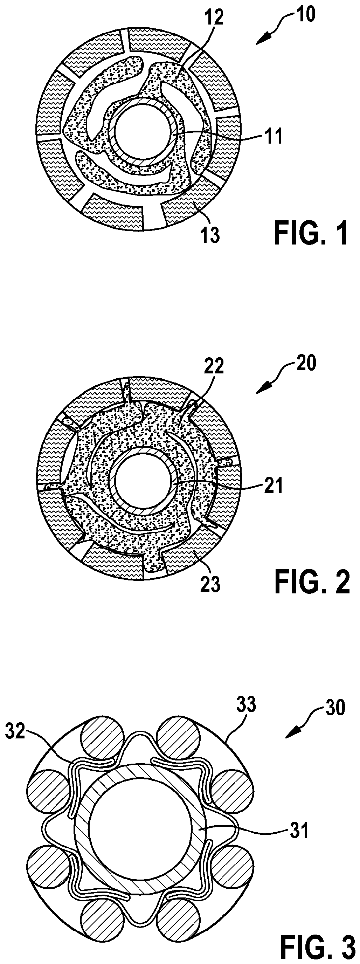

Der Stent wird auf dem Ballonkatheter, speziell über dessen Ballon, typischerweise durch Crimpen befestigt. Fig. 1 zeigt als schematische Querschnittsdarstellung durch den Ballon-Abschnitt eine bekannte Stent-Einführanordnung 10, von der in der Figur ein Schaft 11, ein Ballon 12 und ein Stent 13 zu sehen sind. Der Stent 13 ist ohne besondere zusätzliche Vorkehrungen hinsichtlich der Ballonkonfiguration und Relativposition zwischen Ballon und Stent auf den Ballon 12 aufgecrimpt.

Ein Kraftschluss ist in dieser Form kaum vorhanden. Ein Formschluss wird auch nicht erzeugt. Das Instrument mit gefaltetem Ballon ist zu klein, als dass sich der Stent auf ihm festklammern könnte. Die Stent-Struts gehen beim Crimpen auf Block, d.h. der Crimpdurchmesser kann nicht weiter verkleinert werden, ohne zu riskieren, dass Struts nach innen ausweichen und der Stent deformiert wird, oder Stent-Struts brechen. Nach dem Crimpen vergrößert der Recoil des Stents den Hohlraum zusätzlich. Beim einfachen Crimpen ist die Nominalkraft zwischen Stent und Ballon nicht definiert und kontrolliert. Eine prozessfähige Stentabzugskraft kann deswegen nicht erzielt werden.

Um den erwähnten Nachteilen zu begegnen, wird in der US 6,629,350 eine Stent-Einführanordnung vorgeschlagen, wie sie schematisch im Querschnitt in Fig. 2 gezeigt ist. Auch von dieser Stent-Einführanordnung 20 sind ein Katheterschaft 21, ein Ballon 22 und ein Stent 23 gezeigt. Es ist zu erkennen, dass Ballonseiten in den Zwischenräumen zwischen den einzelnen Stent-Struts eingeklemmt sind, wodurch ein gewisser Formschluss zwischen den Ballon 22 und dem Stent 23 gegeben ist.

Dazu wird der Stent auf den gefalteten Ballon vorgecrimpt und in den Crimpkopf eingelegt. Der irisblendenartige Crimpkopf wird auf einen ersten Innendurchmesser verkleinert und der Ballon mit Druck beaufschlagt, so dass sich Stent und Ballon bis zu diesem Durchmesser öffnen. Bei diesem ersten Durchmesser entfernen sich die Struts voneinander, so dass die dünne Ballonmembran in die entstehenden größeren Stentstrutzwischenräume eindringen kann. In einem zweiten Prozessschritt wird nun unter beibehaltenem Balloninnendruck der Innendurchmesser des Crimpkopfs auf den Zieldurchmesser des gecrimpten Stents verkleinert, so dass die Ballonfalten in den Stentzwischenräumen verbleiben. Der Ballon wird druckentlastet und erst dann der Crimpkopf geöffnet.

Bei kalter Crimpung bleiben die Ballonkonen auf einem Durchmesser, der über dem gecrimpten Durchmesser des Stents liegt. Dies führt dazu, dass der Ballon die Stentenden aufweitet, so dass sich ein wannenartiges Stenprofil ergibt. Die Crimpdurchmesser sind in der Regel größer als bei anderen Verfahren. Dieses sog. Fishscaling an den Stentenden ist nachteilig für die Stenthaltekraft und limitiert den erreichbaren kleinsten gecrimpten Stentdurchmesser. Methodenbedingt wird der Ballon beim Crimpen auf den zweiten Durchmesser zwischen den Struts eingequetscht, was gehäuft Pinholes durch abgeschertes Ballonmaterial verursacht.

Aus der US 6,159,229 ist eine weitere Lösung der eingangs genannten Probleme bekannt, die (wiederum als schematische Querschnittsdarstellung) in Fig. 3 gezeigt ist. Diese Figur zeigt eine Stent-Einführanordnung 30, von der wiederum der Katheterschaft 31, der Ballon 32 und der Stent 33 dargestellt sind, in einem "eingefrorenen" Zwischenzustand mit geringfügig inflatiertem Ballon, vor der Einführung.

Hierfür wird der Stent auf den Ballon vorgecrimpt und dann in eine Röhre des Zielcrimpdurchmessers eingelegt. Der Ballon wird unter Druck gesetzt, und die Röhre wird auf bis zu 100°C erwärmt, um das Ballonmaterial erweichen zu lassen. Der Balloninnendruck bewirkt nun, dass der Ballon die Kontur des Stents annimmt.

Die warme Crimpmethode hat wichtige Nachteile. Die hohe Temperatur sorgt dafür, dass die Ballonmembran sich in Umfangsrichtung des Ballons stark schrumpft, da die Temperatur ohne Formzwang wirkt. Der geschrumpfte Ballondurchmesser wächst bei Druckbelastung wieder in größerem Masse, was die Compliance des Ballons erhöht. Dieser Verlust an Non-Compliant-Behaviour ist für Angioplastieballone nicht erwünscht, zumal die Compliance beim Wiederholen der Dilatation sich nochmals verändert zu flacherer Compliance, aber größeren Startdurchmessern des Ballons. Noch wichtiger ist jedoch, dass bei den relativ hohen Bearbeitungstemperaturen bei den meisten therapeutischen Wirkstoffen ein Abbau zu erwarten ist. Des Weiteren ist ein Risiko vorhanden, dass der erweichte Ballon an der polymeren Stentbeschichtung so stark anhaftet, so dass es beim Freisetzen des Stents zum Ablösen der Stentbeschichtung kommt.

US 6,077,273 offenbart ein Angioplastie- und Stent-Delivery System zur Einführung und Positionierung eines Stents. Der Katheter weist einen distalen Bereich auf, der seinen Durchmesser von einem komprimierten Zustand in einen expandierten Zustand vergrößern kann. Der Stent ist im distalen Bereich des Katheters positioniert.

Der Erfindung liegt die Aufgabe zugrunde, eine verbesserte Stent-Einführanordnung bereit zu stellen, die insbesondere sehr wenig defektanfällig ist und zuverlässig funktioniert. Diese Aufgabe wird durch eine Stent-Einführanordnung mit den Merkmalen des Anspruchs 1 gelöst. Zweckmäßige Fortbildungen des Erfindungsgedankens sind Gegenstand der abhängigen Ansprüche. Es wird des Weiteren ein Ballonkatheter mit den Merkmalen des Anspruchs 6 bereitgestellt.

Die Erfindung geht von folgenden grundsätzlichen Überlegungen aus:
- Die Stentfixierung muss bei möglichst niedriger Temperatur vorzugsweise bei Raumtemperatur erfolgen.
- Die Stentfixierungsmethode muss ausschließen, dass Ballonfalten beim Crimpen des Stents eingequetscht werden können.
- Die Stenthaltekraft auf dem Ballon muss genügend groß sein, dass der Stent gegen Verschieben gesichert ist.

Ausgehend von diesen Grundgedanken, wird eine Anordnung vorgeschlagen, bei der unter dem Ballon ein Federelement vorgesehen ist. Entsprechend ist bei dem Ballonkatheter zur Bildung dieser Stent-Einführanordnung unter dem Ballon ein Federelement vorgesehen, welches eine radial oder schräg zur Längsachse wirkende Federkraft in einem vorbestimmten Wertebereich entwickelt.

Eine aus Anwendungs-Sicht bevorzugte Ausführung zeichnet sich dadurch aus, dass das Federelement derart biegeweich ausgeführt ist, dass es die Biegesteifigkeit der Stent-Einführanordnung nicht wesentlich erhöht. Eine hohe Biegsamkeit der Einführanordnung ist eine wichtige Voraussetzung für umfassende Praxistauglichkeit der Anordnung. In diesem Zusammenhang ist es auch wichtig, dass das Federelement nach innen nachgeben kann, so dass die Stent-Struts sich nicht aufrichten.

In einer weiteren Ausführung der Erfindung ist das Federelement aus einem Abschnitt eines Ballonkatheter-Außenschaftes gebildet, der insbesondere aus einem extrudierten Kunststoff besteht.

In einer Ausgestaltung ist hierbei das Federelement durch einen gewendelten Abschnitt des Ballonkatheter-Außenschaftes gebildet, der durch Erzeugung einer gewendelten Ausnehmung in einem rohrförmigen Außenschaft entsteht. Noch spezieller ist der gewendelte Abschnitt des Ballon-Außenkatheters doppelt gewendelt. Der Außenschaft wird hierzu in den Ballon hinein bis zur Spitze verlängert, damit der Stent beim Crimpen eine definierte Gegenkraft durch Ballon und Außenschaft erfährt.

Als Schaftmaterialien können alle im Katheterbau bekannten biokompatiblen Werkstoffe eingesetzt werden. Vorteilhaft sind Werkstoffe, die sich zu Rohren extrudieren lassen und die sich gut zu Spiralen schneiden lassen.

Eine zu üblichen Kathetern vergleichbar gute Trackability wird erhalten, indem der Schaft im Ballon doppelspiralig geschnitten wird. Durch diesen doppelspiraligen Schnitt wird auch sichergestellt, dass der Ballon trotzdem sicher gefüllt und entleert werden kann. Diese Geometrie ist insofern vorteilhaft, als sie mit einfachsten Mitteln hergestellt werden kann und sehr kostengünstig ist.

Alternativ oder auch in Kombination mit der vorgenannten Ausführung kann ein separates Federelement unter dem Ballon eingesetzt sein. In einer Ausgestaltung dieser Ausführung ist vorgesehen, dass das separate Federelement als Schraubenfederelement aus Metall oder Kunststoff ausgebildet ist.

In einer weiteren Ausführung ist vorgesehen, dass das oder ein Federelement durch einen weichen Innenschaft oder Innenschaftabschnitt des Ballonkatheters gebildet ist. Ein sehr weicher Innenschaft, z.B. aus TPU, der beim Innendurchmesser eine genügend große Spaltbreite zum Führungsdraht hat, kann auch eine definierte radiale Kraft aufnehmen. Diese Lösung ist aus derzeitiger Sicht jedoch nicht optimal, da ohne zusätzliche axiale Verstärkungselement mit einer schlechten Übertragung der Kraft auf die Katheterspitze zu rechnen ist.

Die vorgeschlagene Lösung bietet, zumindest in speziellen Ausführungen, einen oder mehrere der folgenden Vorteile:
- Temperaturempfindliche Materialien und Wirkstoffe können jetzt für das Stent Delivery System verwendet werden.
- Es gibt ein geringeres Fishscaling der Stents, da das Federelement auch Druckspitzen abbaut.
- Die Compliance der Ballone bleibt flacher als bei Hochtemperaturcrimpverfahren.
- Die Stenthaltekraft ist durch gleichmäßige Vorspannung erhöht.

Vorteile und Zweckmäßigkeiten der Erfindung ergeben sich im Übrigen aus der nachfolgenden skizzenartigen Beschreibung von Ausführungsbeispielen anhand der Figuren. Von diesen zeigen:
- Fig. 1: eine Querschnittsdarstellung einer bekannten Stent-Einführanordnung,
- Fig. 2: eine Querschnittsdarstellung einer bekannten weiteren StentEinführanordnung,
- Fig. 3: eine Querschnittsdarstellung einer bekannten weiteren Stent-Einführanordnung,
- Fig. 4: eine Querschnittsdarstellung einer Stent-Einführanordnung gemäß einer Ausführung der Erfindung,
- Fig. 5: eine schematische Seitenansicht einer Stent-Einführanordnung gemäß einer Ausführung der Erfindung und
- Fig. 6: eine Abwicklung eines zur Herstellung einer Ausführungsform der Erfindung bearbeiteten Außenschaft-Abschnittes eines Ballonkatheters.

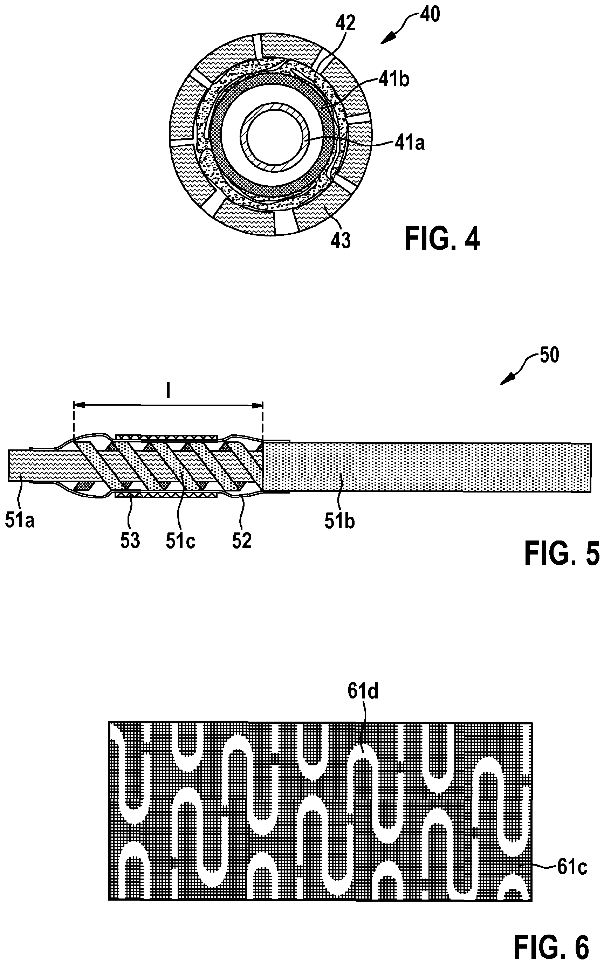

Fig. 4 und 5 zeigen in einer schematischen Querschnittsdarstellung bzw. Seitenansicht, mit an die Bezeichnungsweise der Figuren 1 bis 3 angelehnten Bezeichnungen der Teile, als Ausführungsform der Erfindung eine Stent-Einführanordnung 40 bzw. 50. Es ist zu erkennen, dass der zugehörige (nicht gesondert bezeichnete) Ballonkatheter einen Innenschaft 41a bzw. 51a und einen Außenschaft 41b bzw. 51b aufweist und der Stent 43 bzw. 53 auf einem Abschnitt des Außenschaftes 41b bzw. 51b über dem Ballon 42 bzw. 52 angeordnet ist. Wie in Fig. 5 gezeigt, ist der Außenschaft um einen Abschnitt 1 verlängert, der sich vom proximalen Ende des Ballons bis in dessen distalen Konusbereich hinein erstreckt. Dort ist aus dem Außenschaft ein Wendelabschnitt 51c geformt, der als Federelement im Sinne der Erfindung wirkt.

Der Außenschaft kann z.B. aus dem Material Grilamid L25 bestehen und einen Außendurchmesser von 0,84 mm und einen Innendurchmesser von 0,72 mm haben. Die Steigung der aus dem Außenschaft ausgeschnittenen Wendel kann 2 mm und die Schlitzbreite 0,5 mm betragen.

Fig. 6 zeigt anhand einer Abwicklung des Außenschaftes in die Ebene, wie durch Ausschnitte 61d in das Material des Außenschaftes ein Wendelabschnitt 61c als Federelement gebildet sein kann.

Die Ausführung der Erfindung ist nicht auf die oben beschriebenen Beispiele und hervorgehobenen Aspekte beschränkt, sondern ebenso in einer Vielzahl von Abwandlungen möglich, die im Rahmen fachgemäßen Handelns liegen.

## Patentansprüche

1. Stent-Einführanordnung (40, 50) mit einem Ballonkatheter als Tragstruktur, der einen Ballon (42, 52) umfasst, und einem hierauf mit einer Crimp-Verbindung fixierten Stent (43, 53), wobei im Ballonkatheter unter dem Ballon (42, 52) ein Federelement vorgesehen ist, welches derart ausgebildet ist, dass zwischen Ballon (42, 52) und Stent (43, 53) eine Haltekraft in einem vorbestimmten Wertebereich besteht, **dadurch gekennzeichnet, dass**
das Federelement aus einem Abschnitt (51c) eines Ballonkatheter-Außenschaftes (51b) gebildet ist und
das Federelement durch einen gewendelten Abschnitt (51c) des Ballonkatheter-Außenschaftes (51b) gebildet ist, der durch Erzeugung einer gewendelten Ausnehmung in einem rohrförmigen Außenschaft (51b) entsteht.

2. Stent-Einführanordnung (40, 50) nach Anspruch 1, wobei das Federelement derart biegeweich ausgeführt ist, dass es die Biegesteifigkeit der Stent-Einführanordnung (40, 50) erhöht.

3. Stent-Einführanordnung (40, 50) nach Anspruch 1 oder 2, wobei das Federelement aus einem Abschnitt (51c) eines Ballonkatheter-Außenschaftes (51b) gebildet ist, der aus einem extrudierten Kunststoff besteht.

4. Stent-Einführanordnung (40, 50) nach einem der vorangehenden Ansprüche, wobei der gewendelte Abschnitt (51c) des Ballon-Außenkatheters (51b) doppelt gewendelt ist.

5. Stent-Einführanordnung (40, 50) nach einem der vorangehenden Ansprüche, wobei das Federelement sich bis in einen distalen Konusbereich des Ballons (42, 52) erstreckt.

6. Ballonkatheter zur Bildung einer Stent-Einführanordnung nach einem der vorangehenden Ansprüche, wobei unter einen Ballon ein Federelement vorgesehen ist, welches eine radial oder schräg zur Längsachse wirkende Federkraft in einem vorbestimmten Wertebereich entwickelt, **dadurch gekennzeichnet, dass** das Federelement aus einem Abschnitt (51c) eines Ballonkatheter-Außenschaftes (51b) gebildet ist und
das Federelement durch einen gewendelten Abschnitt (51c) des Ballonkatheter-Außenschaftes (51b) gebildet ist, der durch Erzeugung einer gewendelten Ausnehmung in einem rohrförmigen Außenschaft (51b) entsteht.

## Claims

1. A stent insertion system (40, 50) including a balloon catheter as a supporting structure, which comprises a balloon (42, 52) and a stent (43, 53) fixed thereto by way of a crimp connection, a spring element which is designed in such a way that a holding force within a predefined value range is present between the balloon (42, 52) and the stent (43, 53), the spring element being provided beneath the balloon (42, 52) in the balloon catheter,
**characterized in that**
the spring element is formed of a section (51c) of a balloon catheter outer shaft (51b), and
the spring element is formed by a coiled section (51c) of the balloon catheter outer shaft (51b), which is created by generating a coiled cut-out in a tubular outer shaft (51b).

2. The stent insertion system (40, 50) according to claim 1, wherein the spring element is designed to be so pliable that it increases the flexural rigidity of the stent insertion system (40, 50).

3. The stent insertion system (40, 50) according to claim 1 or 2, wherein the spring element is formed of a section (51c) of a balloon catheter outer shaft (51b) made of an extruded plastic material.

4. The stent insertion system (40, 50) according to any one of the preceding claims, wherein the coiled section (51c) of the balloon catheter outer shaft (51b) is double-coiled.

5. The stent insertion system (40, 50) according to any one of the preceding claims, wherein the spring element extends into a distal cone region of the balloon (42, 52).

6. A balloon catheter for forming a stent insertion system according to any one of the preceding claims, a spring element which develops a spring force acting radially or obliquely with respect to the longitudinal axis within a predefined value range being provided beneath a balloon, **characterized in that**
the spring element is formed of a section (51c) of a balloon catheter outer shaft (51b), and
the spring element is formed by a coiled section (51c) of the balloon catheter outer shaft (51b), which is created by generating a coiled cut-out in a tubular outer shaft (5 1b).

## Revendications

1. Ensemble d'insertion d'endoprothèse (40, 50) doté d'un cathéter à ballonnet en tant que structure support, qui comprend un ballonnet (42, 52), et une endoprothèse (43, 53) fixée sur celui-ci avec une connexion par sertissage, où un élément ressort est prévu dans le cathéter à ballonnet en-dessous du ballonnet (42, 52), lequel est conçu de telle manière qu'une force de maintien qu'il existe une plage de valeurs prédéfinie entre le ballonnet (42, 52) et l'endoprothèse (43, 53),
**caractérisé en ce que**
l'élément ressort est formé à base d'un segment (51c) d'une tige extérieure de cathéter à ballonnet (51b), et
l'élément ressort est formé par une section à enroulements (51c) de la tige extérieure de cathéter à ballonnet (51b) qui se crée par la génération d'une cavité à enroulements dans une tige extérieure (51b) en forme de tube.

2. Ensemble d'insertion d'endoprothèse (40, 50) selon la revendication 1, dans lequel l'élément ressort est conçu souple en flexion dans une telle mesure qu'il augmente la résistance en flexion de l'ensemble d'insertion d'endoprothèse (40, 50).

3. Ensemble d'insertion d'endoprothèse (40, 50) selon la revendication 1 ou 2, dans lequel l'élément ressort est formé à partir d'un segment (51c) d'une tige extérieure de cathéter à ballonnet (51b) qui est constitué d'une matière plastique extrudée.

4. Ensemble d'insertion d'endoprothèse (40, 50) selon l'une des revendications précédentes, dans lequel le segment à enroulements (51c) du cathéter extérieur à ballonnet (51b) est à double enroulement.

5. Ensemble d'insertion d'endoprothèse (40, 50) selon l'une des revendications précédentes, dans lequel l'élément ressort s'étend jusqu'à une zone en cône distale du ballonnet (42, 52).

6. Cathéter à ballonnet destiné à la formation d'un ensemble d'insertion d'endoprothèse selon l'une des revendications précédentes, où un élément ressort est prévu en-dessous d'un ballonnet, lequel développe une force de rappel dans une plage de valeurs prédéterminée agissant radialement ou en biais par rapport à l'axe longitudinal, **caractérisé en ce que**
l'élément ressort est formé à base d'un segment (51c) d'une tige extérieure de cathéter à ballonnet (51b) et
l'élément ressort est formé par un segment à enroulements (51c) de la tige extérieure de cathéter à ballonnet (51b) qui se crée par la génération d'une cavité à enroulements dans une tige extérieure (51b) en forme de tube.
